(19) Europäisches Patentamt
European Patent Office
Office européen des brevets

(11) **EP 3 444 771 A1**

(12) **EUROPEAN PATENT APPLICATION**

(43) Date of publication:
**20.02.2019 Bulletin 2019/08**

(51) Int Cl.:
**G06Q 40/04** (2012.01)  **G06Q 10/06** (2012.01)

(21) Application number: **18183285.8**

(22) Date of filing: **13.07.2018**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN**<br><br>(30) Priority: **18.08.2017 CN 201710714345**<br>**18.08.2017 CN 201710713805**<br><br>(71) Applicant: **Hepu Technology Development (Beijing) Co. Ltd.**<br>**100176 Beijing (CN)** | (72) Inventors:<br>• **Cui, Hua**<br>  **Beijing (CN)**<br>• **Yang, Yusen**<br>  **Beijing (CN)**<br>• **Xu, Bo**<br>  **Beijing (CN)**<br>• **Tan, Zhi**<br>  **Beijing (CN)**<br>• **Chen, Hui**<br>  **Beijing (CN)**<br><br>(74) Representative: **Huang, Liwei**<br>**Cäcilienstraße 12**<br>**40597 Düsseldorf (DE)** |

(54) **BLOCKCHAIN-BASED CARBON TRADING SYSTEM**

(57) The present invention discloses a blockchain-based carbon trading system, relates to the technical field of greenhouse gas monitoring and block chains. The carbon trading system comprises: a carbon emission measurement module (10), a block chain carbon emission checking module (20), a carbon emission reduction calculation module (30), and a block chain carbon emission reduction checking module (40). The carbon emission measurement module (10) is configured to measure carbon emission of greenhouse gas to generate carbon emission data; the block chain carbon emission checking module (20) is configured to add a timestamp onto the carbon emission data to generate carbon emission information; the carbon emission reduction calculation module (30) is configured to measure carbon emission reduction of reduced greenhouse gas to generate carbon emission reduction data; and the block chain carbon emission reduction checking module (40) is configured to add a timestamp onto the carbon emission reduction data to check the generated carbon emission reduction information. Through the system provided by the present invention, a carbon emission calculation error is reduced, and the credibility and the trading efficiency of an existing carbon trading market system and green certificate trading system are improved.

FIG. 1

EP 3 444 771 A1

**Description**

**FIELD OF THE INVENTION**

The present invention relates to the technical field of greenhouse gas monitoring and block chains, and more particularly, to a blockchain-based carbon trading system.

**BACKGROUND OF THE INVENTION**

During China's "13th Five-year Plan" period for 2016-2020, it is predicted that stressing energy conservation and emission reduction, developing energy-saving and environmentally friendly industries and boosting reforms of institutional mechanisms for ecological civilization will continue to be the focus of development. In the "13th Five-year Plan", various plans are proposed to elaborate trading mechanisms for energy and environmental property rights, so as to establish and improve an initial distribution system for energy use rights, water use rights, pollutant emission rights and carbon emission rights, speed up the establishment of the electricity market by cultivating the auxiliary electricity service market and setting up a renewable energy quota system and a green electricity certificate trading system, and carry out energy use right trading pilot projects to promote the establishment of a unified carbon emission trading market in China.

Establishing a domestic carbon trading system is an important way to control China's growing greenhouse gas emissions. It is also a need for China to adjust energy and industrial structure, improve energy efficiency, guide investment and technology flows,. At present, in view of insufficient energy resources and severe air pollution, China adheres to the guiding principle of the decisive role of the market in resource allocation, so establishing a carbon trading system is a strategic measure that uses market means to promote energy conservation, emission reduction and environmental protection. Compared with other trading systems, pilot work of the carbon trading system in seven provinces/municipalities in China had been advanced rapidly. In March 2011, China's "12th Five-year Plan" for 2010-2015 proposed to gradually establish a carbon trading market. From 2013 to June 2014, seven provinces/municipalities had started the carbon emission right trading market successively. By the end of December 31, 2016, China's domestically traded quota volume reached 86.7 million tons with a turnover of RMB 2 billion.

On February 3, 2017, the National Development and Reform Commission of China, China's Finance Ministry, and the National Energy Administration of China jointly issued the "Notice on Trial Implementation of Renewable Energy Green Electricity Certificate Verification and Issuance and Voluntary Subscription Trading System" (No. [2017]-132 for energy issued by the NDRC) to pilot the verification, issuance and voluntary subscription of a renewable energy green electricity certificate (hereinafter referred to as "green certificate") nationwide. The "green certificate" trading policy was a means to replace the existing subsidies for new energy power generation companies. It would relieve the pressure of state financial subsidies to a certain extent, and in particular help ease the current situation of subsidies arrears.

The Paris Agreement entered into force on November 4, 2016. On that day, the State Council of China issued the "13th Five-year Greenhouse Gas Emission Control Program, explicitly proposing an aim of "controlling the carbon dioxide emission per unit power supply of large-scale power generation groups to be within 550 g $CO_2$/ kWh". In the future, China will formulate carbon dioxide emission quota requirements on other high energy-consuming enterprises such as the chemical industry, the iron and steel industry and the cement industry. If thermal power plants or high energy-consuming enterprises want to maintain their production scale, they need to purchase a non-hydraulic renewable energy "green certificate" or a carbon emission quota of the carbon emission trading right to reach power generation or production quota assessment indicators, so as to ultimately achieve the macro goal that China's non-fossil energy consumption will account for 15% of the primary energy consumption in 2020.

Under such a background, carbon emission monitoring and carbon emission right trading of big carbon emitters, such as fossil fuel power plants or other large-scale high energy-consuming fossil fuel boilers, become more and more important. Currently, there is no on-line monitoring methods for carbon dioxide emissions for most thermal power plants and high energy-consuming boilers. The carbon dioxide emission amounts of many thermal power plants or coal-fired boilers are indirectly calculated based on the coal consumption amounts through a carbon balance method or an emission factor method, but these data cannot be used as basis for determining the actual carbon emission amounts of large-scale fossil energy boilers. In addition, the existing carbon trading market system and green certificate trading system are inefficient due to a complicated carbon emission checking process, and the credibility of the trading data is being questioned . Thus, the trading volume and establishment effect of these two systems cannot meet China's requirements for energy conservation and emission reduction as well as the Paris Agreement requirements.

In November 2008, a scientist, called Satoshi Nakamoto, published a paper entitled as "Bitcoin: A Peer-to-Peer Electronic Cash System". The paper proposed a concept for establishing an electronic cash system based on the P2P networking technology, mathematics, the cryptography technology, algorithms and economic modeling, and developed the genesis block with the serial number of 0 for that system in January 2009, creating the Bitcoin. However, at that time, people did not concern the concept of block chain. People started to mine the underlying technology that supports the Bitcoin system-the block chain technology until the Bitcoin system was safely operated without professional maintenance personnel for many years. Block chain refers to a technical solution for collectively maintaining a reliable database through decentralization and non-delegation. Through this technical solution, any node in the system may generate a data block through a cryptography-related algorithm. Each data block comprises two parts, namely a block head and block data. The block head comprises external information such as a version number, a hash value, a difficulty value, a Mercle tree and Nounce which are configured to verify the validity of the information and chain a next data block. The block data contains all trading data information of the system within a certain period of time.

The block chain technology can be used to combat fraud and illegal trading, and many industries are using the block chain technology. Particularly, the energy internet technologies are also using the block chain technology as a tool. The block chain technology has the following functions. First, the fair blockchain-based data ensures credibility, and the combined public and private key access permission protects privacy, realizing complete privacy and credible calculation. Second, the block chain technology prevents falsification as subjects adopt a certain way to realize cooperative or compulsory trust, so as to achieve ubiquitous interaction under compulsory trust. Third, the block chain, big data and artificial intelligence technologies are combined to form a trustworthy oracle model to verify external data, so that self-discipline of virtual reality interaction is realized. Fourth, devices allocated based on the block chain technology make decisions in a peer-to-peer interactive manner, and do not need to entrust a centralized platform to make decisions, so that device democracy and distributive decision-making are realized by decentralization. Fifth, the subjects carry out stochastic game based on clear interaction rules, so that the system presents a neutral or benign evolution, thereby meeting the marketization law as well as the coordination and evolvability in competition evolution.

The block chain technology, as the underlying technology of the crypto currency Bitcoin, is a great innovation. The combination of the block chain technology with carbon emission monitoring, the carbon trading market and the green certificate can greatly improve the credibility and the trading efficiency of the existing carbon trading market system and green certificate trading system.

## SUMMARY OF THE INVENTION

An object of the present invention is to improve the credibility and the trading efficiency of an existing carbon trading market system and green certificate trading system.

In order to achieve the object of the present invention, according to one aspect of the present invention, there is provided a blockchain-based carbon trading system, comprising: a carbon emission measurement module configured to measure carbon emission of greenhouse gas produced by fossil fuel burning or gas leakage in the fields of energy, industry and/or transportation to generate carbon emission data; a block chain carbon emission checking module configured to receive the carbon emission data generated by the carbon emission measurement module and add a timestamp onto the carbon emission data to generate carbon emission information; a carbon emission reduction calculation module configured to measure carbon emission reduction of reduced greenhouse gas in the fields of energy, industry and/or transportation after green energy and energy-saving measures are adopted to generate carbon emission reduction data; and a block chain carbon emission reduction checking module configured to receive the carbon emission reduction data generated by the carbon emission reduction calculation module and add a timestamp onto the carbon emission reduction data to check the generated carbon emission reduction information.

Further, in the blockchain-based carbon trading system, the carbon emission measurement module is further configured to measure a greenhouse gas content of flue gas discharged from boilers in the field of energy, and obtain carbon emission data of the flue gas based on the measured greenhouse gas content.

Further, in the blockchain-based carbon trading system, the carbon emission measurement module is further configured to measure a content of greenhouse gas discharged from steel-making furnaces or boilers of cement production lines in the field of industry or a content of greenhouse gas leaked by petrochemical and refining industries, and obtain carbon emission data in the field of industry based on the measured greenhouse gas content.

Further, in the blockchain-based carbon trading system, the carbon emission measurement module is further configured to measure a greenhouse gas content of exhaust gas emitted by vehicles in the field of transportation, and calculate carbon emission data of the vehicles through mileage and fuel consumption of the vehicles.

Further, in the blockchain-based carbon trading system, the carbon emission measurement module comprises a carbon dioxide monitoring module and a comprehensive carbon dioxide emission monitoring and calculation module. The carbon dioxide monitoring module is configured to monitor a carbon dioxide concentration of flue gas, and transmit the obtained carbon dioxide concentration data to the comprehensive carbon dioxide emission monitoring and calculation module. The comprehensive carbon dioxide emission monitoring and calculation module is configured to obtain carbon dioxide emission data of the flue gas according to the received carbon dioxide concentration data and pre-monitored total flue gas flow data.

Further, in the blockchain-based carbon trading system, the carbon emission measurement module further comprises a module for monitoring other greenhouse gases and a carbon dioxide conversion module. The module for monitoring other greenhouse gases is configured to monitor a concentration of other greenhouse gases except carbon dioxide in the flue gas, and transmit the obtained concentration data of other greenhouse gases to the carbon dioxide conversion module. The carbon dioxide conversion module is configured to obtain equivalent carbon dioxide concentration data through conversion according to the concentration data of other greenhouse gases, and transmit the converted carbon dioxide concentration data to the comprehensive carbon dioxide emission monitoring and calculation module. The comprehensive carbon dioxide emission monitoring and calculation module is configured to calculate carbon emission data of the flue gas according to the received carbon dioxide concentration data, pre-monitored total flue gas flow data and the converted carbon dioxide concentration data.

Further, in the blockchain-based carbon trading system, the carbon dioxide monitoring module is connected to a flue gas on-line sampling device to monitor a carbon dioxide concentration of the flue gas through at least one of a non-dispersive infra-red (NDIR) analysis method, a tunable diode laser absorption spectroscopy (TDLAS) method, an infrared spectroscopy method, a gas sensitive electrode method, a gas chromatography method, a gas filter monitoring method, and a wavelength scanning-cavity ring-down spectroscopy (WS-CRDS) method.

Further, in the blockchain-based carbon trading system, the module for monitoring other greenhouse gases is connected to the flue gas on-line sampling device to monitor a concentration of other greenhouse gases except carbon dioxide in the flue gas through at least one of the non-dispersive infra-red (NDIR) analysis method, the tunable diode laser absorption spectroscopy (TDLAS) method, the infrared spectroscopy method, the gas sensitive electrode method, the gas chromatography method, the gas filter monitoring method, and the wavelength scanning-cavity ring-down spectroscopy (WS-CRDS) method.

Further, in the blockchain-based carbon trading system, the module for monitoring other greenhouse gases comprises a methane monitoring component, a nitrous oxide monitoring component and/or a fluoride monitoring component.

Further, in the blockchain-based carbon trading system, the carbon dioxide conversion module adopts at least one of a global warming potential (GWP) conversion method, a global temperature change potential (GTP) conversion method and a comprehensive conversion method to convert the concentration of other greenhouse gases into a carbon dioxide concentration.

Further, in the blockchain-based carbon trading system, the comprehensive conversion method is based on the following equation (1) for calculation: $GWP_{corrected} = A*GWP+B*GTP$ Equation (1).

In the above equation, $GWP_{corrected}$ represents a carbon dioxide concentration converted through the comprehensive conversion method; $GWP$ represents a carbon dioxide concentration converted through the GWP conversion method; the GTP represents a carbon dioxide concentration converted through the GTP conversion method; and A and B represent predetermined correction coefficients, respectively.

Further, in the blockchain-based carbon trading system, the module for monitoring other greenhouse gases comprises one or more of a methane monitoring module, a nitrous oxide monitoring module, a hydrofluorocarbon monitoring module, a perfluorocarbon monitoring module, a hexafluoroethane monitoring module and a nitrogen trifluoride monitoring module.

Further, in the blockchain-based carbon trading system, the block chain carbon emission checking module is further configured to assign each greenhouse gas emission unit in the fields of energy, industry and transportation with a unique ID.

Further, in the blockchain-based carbon trading system, the block chain carbon emission checking module is further configured to add a timestamp onto carbon emission data and a carbon emission right of each greenhouse gas emission unit in the fields of energy, industry and transportation, and record the timestamp in the block chain. The carbon emission data is obtained from the carbon emission measurement module.

Further, in the blockchain-based carbon trading system, the block chain carbon emission checking module adopts a smart contract to automatically determine carbon emission right consumption arising from the carbon emission of each greenhouse gas emission unit.

Further, in the blockchain-based carbon trading system, the block chain carbon emission checking module is further configured to complete carbon emission right trading among different greenhouse gas emission units or between a greenhouse gas emission unit and a carbon emission reduction unit. Trading information generated during each carbon emission right trading is recorded in the block chain and is non-falsifiable.

Further, in the blockchain-based carbon trading system, the carbon emission reduction units comprise related units capable of reducing carbon emissions and summarized based on a CDM methodology of the UNFCCC.

Further, in the blockchain-based carbon trading system, the block chain carbon emission checking module is distributed in each node of a block chain, and is capable of automatically levy a fine on a greenhouse gas emission unit whose carbon emission exceeds a preset carbon emission.

Further, in the blockchain-based carbon trading system, the flue gas comes from a fossil fuel boiler in at least one of a large-scale thermal power plant, a large-scale steel-making plant, a large-scale coking plant, a large-scale cement plant and a large-scale chemical plant.

Further, in the blockchain-based carbon trading system, the block chain carbon emission checking module is provided with a data layer for storing the following data: the carbon emission data measured by the carbon emission measurement module; the carbon emission right trading data which is all peer-to-peer carbon emission right trading data in the block chain; a carbon purchasing and selling identifier which is a binary digit character string for distinguishing a carbon emission right purchaser from a carbon emission right seller, the first binary digit character string in the carbon purchasing and selling identifier representing that a current block chain node is the carbon emission right purchaser, and the second binary digit character string in the carbon purchasing and selling identifier representing that a current block chain node is the carbon emission right seller; and the timestamp for recording the peer-to-peer carbon emission right trading time in a block chain network. The data layer is further configured to add a timestamp onto carbon emission right trading data in each node to form a data block which is recorded in the block chain and is non-falsifiable.

Further, in the blockchain-based carbon trading system, the fossil fuel generating the flue gas comprises at least one of coal, natural gas, coal gas, oil, coke, coal gangue, coal water slurry and blue carbon.

At present, most of the carbon emissions of high energy-consuming enterprises are indirectly calculated based on their coal consumption through a carbon balance method or an emission factor method. Carbon emission reductions of clean energy or energy-saving projects are calculated by using the CDM methodology. These calculated carbon emissions and carbon emission reductions have the problems of large calculation errors and low data reliability. In addition, the existing carbon trading market system and green certificate trading system for carbon assets and carbon emission rights also have the problems of repeated establishment, poor cooperative governance effect, low credibility, low trading efficiency, and high trading and credit cost.

By combining the block chain technology with a greenhouse gas carbon emission monitoring system, the blockchain-based carbon trading system provided by the present invention has the following advantages compared with the prior art.

1. Greenhouse gas on-line or sampling monitoring can solve the problems of large errors, highly possible data statistics disputes and low credibility in a conventional carbon emission calculation method.

2. Uniformly converting emissions of various non-carbon dioxide greenhouse gases into carbon dioxide emissions can facilitate statistics and carbon asset management; and meanwhile, greenhouse gas emissions may be monitored comprehensively.

3. Through the advantages of the block chain technology such as decentralization, transparency, non-falsification, non-forgeability and system self-government, emission data of carbon emission units and emission reduction data of emission reduction units can be stored safely and transparently in the block chain monitoring module, so that multilateral peer-to-peer trading of self-governing carbon assets and carbon emission rights can be realized.

4. By combining the block chain technology with the greenhouse gas on-line or sampling monitoring, real-time, transparent and non-falsifiable block chain carbon asset management for carbon assets and carbon emission rights of high energy-consuming carbon emission enterprises and green carbon emission reduction enterprises can be realized, providing the technical support for carbon finance development in the future.

**BRIEF DESCRIPTION OF THE DRAWINGS**

Fig. 1 is a schematic view of the overall architecture of a blockchain-based carbon trading system;

Fig. 2 is a schematic view of the overall architecture of a blockchain-based carbon trading system in a first embodiment;

Fig. 3 is a schematic view of the overall architecture of a blockchain-based carbon trading system in a second embodiment;

Fig. 4 is a schematic view of the overall architecture of a blockchain-based carbon trading system in a third embodiment;

Fig. 5 is a schematic view of the relationship among modules in a carbon emission measurement module;

Fig. 6 is a schematic view of greenhouse gas sampling, monitoring and analysis methods; and

Fig. 7 is a schematic view of carbon dioxide conversion calculation methods.

**DETAILED DESCRIPTION OF THE EMBODIMENTS**

In order to explain the objects, technical solutions and advantages of the present invention more apparently, the present invention is further described in detail below with reference to the specific embodiments and accompanying drawings. It should be understood that these descriptions are merely exemplary and are not intended to limit the scope of the present invention. In addition, in the following description, descriptions of well-known structures and techniques are omitted to avoid unnecessarily obscuring the concepts of the present invention.

The core of the present invention is to combine the block chain technology with a greenhouse gas carbon emission monitoring system, so as to accurately monitor carbon emissions of large-scale power plant boilers and other high energy-consuming enterprises, and realize carbon emission monitoring and carbon emission right trading in China and even the world.

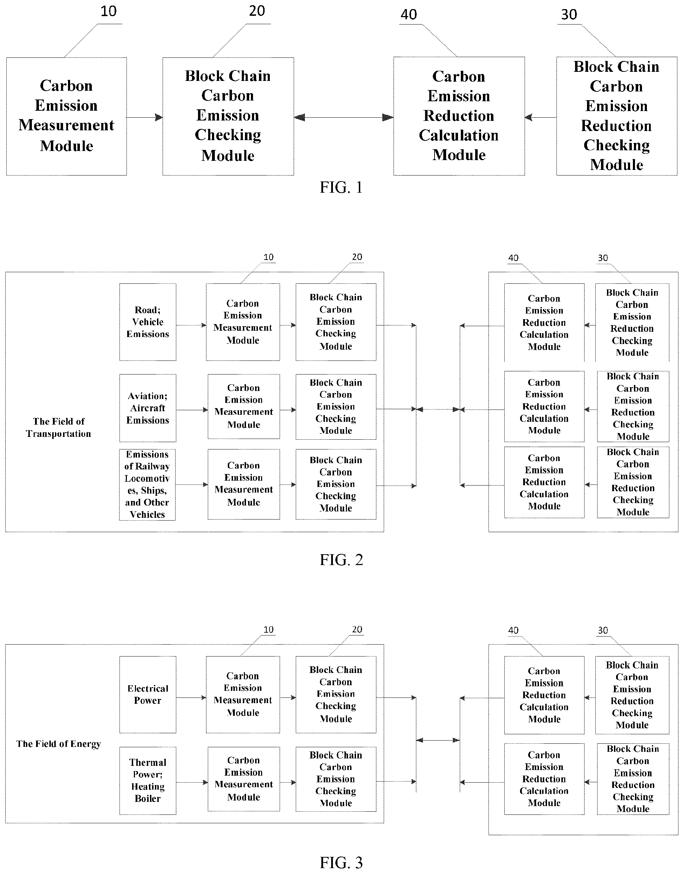

Fig. 1 is a schematic view of the overall architecture of a blockchain-based carbon trading system.

As shown in Fig. 1, the present invention provides a blockchain-based carbon trading system, comprising: a carbon emission measurement module 10, a block chain carbon emission checking module 20, a carbon emission reduction calculation module 30, and a block chain carbon emission reduction checking module 40.

The carbon emission measurement module 10 is configured to measure carbon emission of greenhouse gas produced by fossil fuel burning or gas leakage in the fields of energy, industry and/or transportation to generate carbon emission data, and transmit the carbon emission data to the block chain carbon emission checking module 20.

The block chain carbon emission checking module 20 is configured to receive the carbon emission data generated by the carbon emission measurement module 10 and add a timestamp onto the carbon emission data to generate carbon emission information.

The carbon emission reduction calculation module 30 is configured to measure carbon emission reduction of reduced greenhouse gas in the fields of energy, industry and/or transportation after green energy and energy-saving measures are adopted to generate carbon emission reduction data.

A carbon purchasing and selling identifier is a binary digit character string for distinguishing a carbon emission right purchaser from a carbon emission right seller. The first binary digit character string in the carbon purchasing and selling identifier represents that a current block chain node is the carbon emission right purchaser; and the second binary digit character string in the carbon purchasing and selling identifier represents that a current block chain node is the carbon emission right seller.

Optionally, the binary digit character string may be a single digit or multiple digits.

The first binary digit character string and the second binary digit character string include but are not limited to the following definitions.

Optionally, it is defined that the first binary digit character string 0 represents that the current node is a carbon emission right purchaser, and the second first binary digit character string 1 represents that the current node is a carbon emission right seller, and vice versa. That is, it is defined that the first binary digit character string 1 represents that the current node is the carbon emission right seller, and the second first binary digit character string 0 represents that the current node is the carbon emission right purchaser.

Optionally, it is defined that the first binary digit character string 00 represents that the current node is an electricity purchaser, and the second binary digit character string 11 represents the current node is a carbon emission right purchaser, and vice versa.

Optionally, it is defined that the first binary digit character string 01 represents that the current node is an electricity purchaser, and the second binary digit character string 10 represents the current node is a carbon emission right seller, and vice versa.

The block chain carbon emission reduction checking module 40 is configured to receive the carbon emission reduction data generated by the carbon emission reduction calculation module 30 and add a timestamp onto the carbon emission reduction data to check the generated carbon emission reduction information.

Fig. 2 is a schematic view of the overall architecture of a blockchain-based carbon trading system in a first embodiment.

As shown in Fig. 2, the carbon emission measurement module 10 is configured to measure a greenhouse gas content of flue gas discharged from boilers in the field of energy, and obtain carbon emission data of the flue gas based on the measured greenhouse gas content. The boiler is at least one of a power station boiler related to power generation or a heating boiler related to heating power in the field of energy.

Fig. 3 is a schematic view of the overall architecture of a blockchain-based carbon trading system in a second embodiment.

As shown in Fig. 3, the carbon emission measurement module 10 is configured to measure a content of greenhouse gas discharged from steel-making furnaces or boilers of cement production lines in the field of industry or a content of greenhouse gas leaked by petrochemical and refining industries, and obtain carbon emission data in the field of industry based on the measured greenhouse gas content.

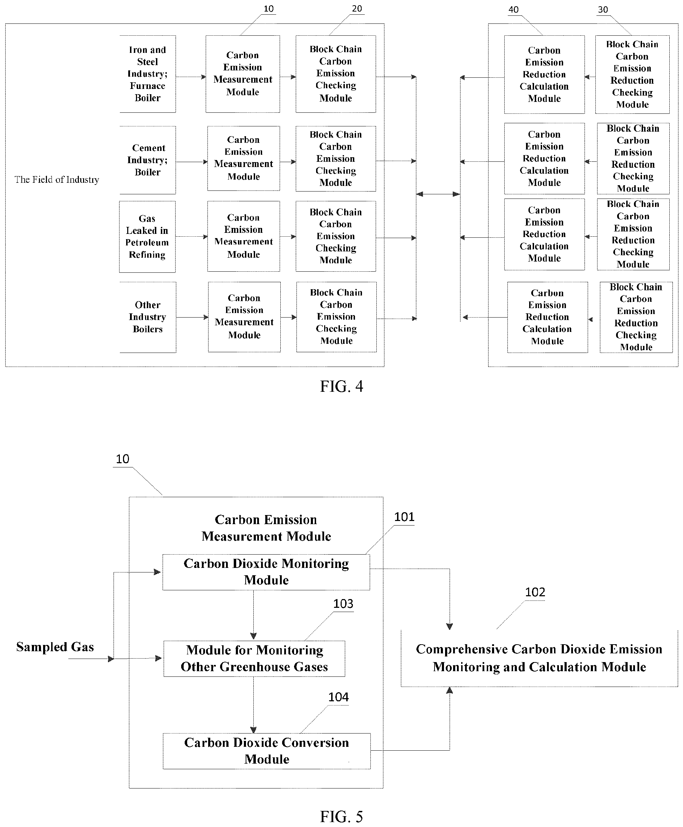

Fig. 4 is a schematic view of the overall architecture of a blockchain-based carbon trading system in a third embodiment.

As shown in Fig. 4, the carbon emission measurement module 10 is configured to measure a greenhouse gas content of exhaust gas emitted by vehicles in the field of transportation, and calculate carbon emission data of the vehicles through mileage and fuel consumption of the vehicles.

Fig. 5 is a schematic view of the relationship among modules in a carbon emission measurement module 10.

As shown in Fig. 5, the carbon emission measurement module 10 comprises a carbon dioxide monitoring module and a comprehensive carbon dioxide emission monitoring and calculation module.

The carbon dioxide monitoring module is configured to monitor a carbon dioxide concentration of flue gas, and transmit the obtained carbon dioxide concentration data to the comprehensive carbon dioxide emission monitoring and calculation module.

The comprehensive carbon dioxide emission monitoring and calculation module is configured to obtain carbon dioxide emission data of the flue gas according to the received carbon dioxide concentration data and pre-monitored total flue gas flow data.

Further, the carbon emission measurement module 10 further comprises a module for monitoring other greenhouse gases and a carbon dioxide conversion module.

The module for monitoring other greenhouse gases is configured to monitor a concentration of other greenhouse gases except carbon dioxide in the flue gas, and transmit the obtained concentration data of other greenhouse gases to the carbon dioxide conversion module. The carbon dioxide conversion module is configured to obtain equivalent carbon dioxide concentration data through conversion according to the concentration data of other greenhouse gases, and transmit the converted carbon dioxide concentration data to the comprehensive carbon dioxide emission monitoring and calculation module.

The comprehensive carbon dioxide emission monitoring and calculation module is configured to calculate carbon emission data of the flue gas according to the received carbon dioxide concentration data, pre-monitored total flue gas flow data and the converted carbon dioxide concentration data.

The carbon dioxide monitoring module is connected to a flue gas on-line sampling device to monitor a carbon dioxide concentration of the flue gas through at least one of a non-dispersive infra-red (NDIR) analysis method, a tunable diode laser absorption spectroscopy (TDLAS) method, an infrared spectroscopy method, a gas sensitive electrode method, a gas chromatography method, a gas filter monitoring method, and a wavelength scanning-cavity ring-down spectroscopy (WS-CRDS) method.

The module for monitoring other greenhouse gases is connected to the flue gas on-line sampling device to monitor a concentration of other greenhouse gases except carbon dioxide in the flue gas through at least one of the NDIR method, the TDLAS method, the infrared spectroscopy method, the gas sensitive electrode method, the gas chromatography method, the gas filter monitoring method, and the WS-CRDS method. The module for monitoring other greenhouse gases comprises a methane monitoring component, a nitrous oxide monitoring component and/or a fluoride monitoring component.

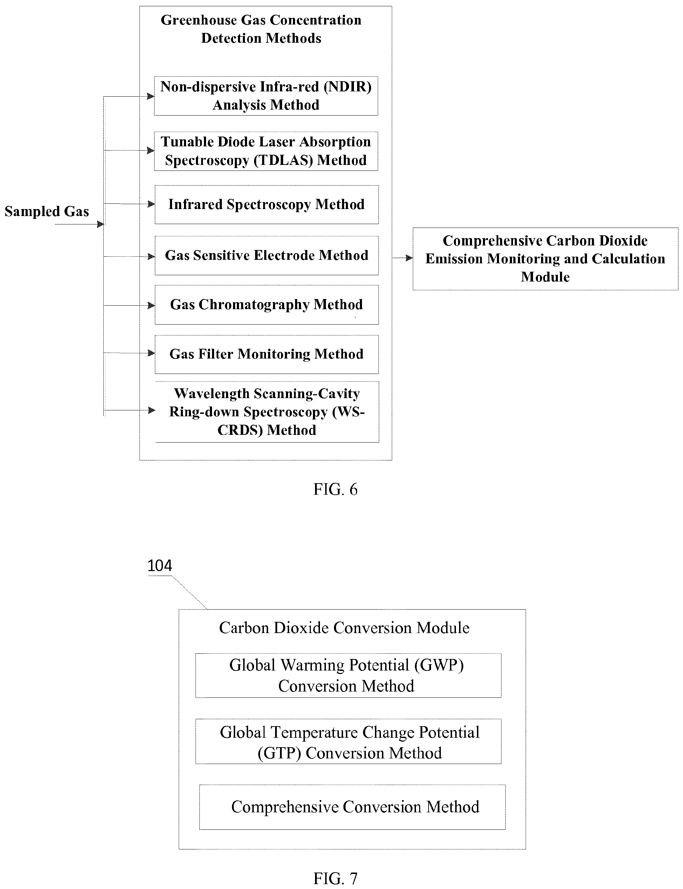

Fig. 7 is a schematic view of carbon dioxide conversion calculation methods.

As shown in Fig. 7, the carbon dioxide conversion module adopts at least one of a global warming potential (GWP) conversion method, a global temperature change potential (GTP) conversion method and a comprehensive conversion method to convert the concentration of other greenhouse gases into a carbon dioxide concentration.

The comprehensive conversion method is based on equation (1) for calculation:

$$GWP_{\text{corrected}} = \text{A*GWP+B*GTP} \qquad\qquad \text{Equation (1).}$$

In the above equation, $GWP_{\text{corrected}}$ represents a carbon dioxide concentration converted through the comprehensive conversion method; GWP represents a carbon dioxide concentration converted through the GWP conversion method; the GTP represents a carbon dioxide concentration converted through the GTP conversion method; and A and B represent predetermined correction coefficients, respectively.

In another embodiment of the present invention, the module for monitoring other greenhouse gases comprises one or more of a methane monitoring module, a nitrous oxide monitoring module, a hydrofluorocarbon monitoring module, a perfluorocarbon monitoring module, a hexafluoroethane monitoring module and a nitrogen trifluoride monitoring module.

In yet another embodiment of the present invention, the block chain carbon emission checking module 20 is further configured to assign each greenhouse gas emission unit in the fields of energy, industry and transportation with a unique ID.

In yet another embodiment of the present invention, the block chain carbon emission checking module 20 is further configured to add a timestamp onto carbon emission data and a carbon emission right of each greenhouse gas emission unit in the fields of energy, industry and transportation, and record the timestamp in the block chain. The carbon emission data is obtained from the carbon emission measurement module 10.

In yet another embodiment of the present invention, the block chain carbon emission checking module 20 adopts a smart contract to automatically determine carbon emission right consumption arising from the carbon emission of each greenhouse gas emission unit.

In yet another embodiment of the present invention, the block chain carbon emission checking module 20 is further configured to complete carbon emission right trading among different greenhouse gas emission units or between a greenhouse gas emission unit and a carbon emission reduction unit. Trading information generated during each carbon emission right trading is recorded in the block chain and cannot be falsified.

In yet another embodiment of the present invention, the carbon emission reduction units comprise related units capable of reducing carbon emissions and summarized based on the CDM (Clean Development Mechanism) methodology of the UNFCCC.

In yet another embodiment of the present invention, the block chain carbon emission checking module 20 is distributed in each node of the block chain, and can automatically levy a fine on a greenhouse gas emission unit whose carbon emission exceeds a preset carbon emission.

In yet another embodiment of the invention, the flue gas comes from fossil fuel boilers in at least one of a large-scale thermal power plant, a large-scale steel-making plant, a large-scale coking plant, a large-scale cement plant and a large-scale chemical plant.

In yet another embodiment of the invention, the fossil fuel generating the flue gas comprises at least one of coal, natural gas, coal gas, oil, coke, coal gangue, coal water slurry and blue carbon.

At present, most of the carbon emissions of high energy-consuming enterprises in China are indirectly calculated based on the coal consumption through a carbon balance method or an emission factor method. Carbon emission reductions of clean energy or energy-saving projects are calculated by using the CDM methodology. These calculated carbon emissions and carbon emission reductions have the problems of large calculation errors and low data reliability. In addition, the existing carbon trading market system and green certificate trading system for carbon assets and carbon emission rights also have the problems of repeated establishment, poor cooperative governance effect, low credibility, low trading efficiency, and high trading and credit cost.

By combining the block chain technology with the greenhouse gas carbon emission monitoring system in the above embodiments, the blockchain-based carbon trading system provided by the present invention has the following advantages compared with the prior art.

1. On-line or sample monitoring of greenhouse gases can solve the problems of large errors, highly possible data statistics disputes and low credibility in a conventional carbon emission calculation method.
2. Uniformly converting emissions of various non-carbon dioxide greenhouse gases into a carbon dioxide emission can facilitate statistics and carbon asset management; and meanwhile, greenhouse gas emission may be monitored comprehensively.
3. Through the advantages of the block chain technology such as decentralization, transparency, non-falsification, non-forgeability and system self-government, emission data of carbon emission units and emission reduction data of emission reduction units can be stored safely and transparently in the block chain monitoring module, so that multilateral peer-to-peer trading of self-governing carbon assets and carbon emission rights can be realized.
4. By combining the block chain technology with the greenhouse gas on-line or sampling monitoring, real-time, transparent and non-falsifiable block chain carbon asset management for carbon assets and carbon emission rights of high energy-consuming carbon emission enterprises and green energy-saving carbon emission reduction enterprises can be realized, providing the technical support for carbon finance development in the future.

The above describes the flue gas and greenhouse gas emission monitoring system provided by the present invention in detail. The principles and implementations of the present invention are illustrated through the specific embodiments, and the above description of the embodiments is only used to help understand the methods and core concept of the present invention. It should be noted that those skilled in the art can make various improvements and modifications to the present invention without departing from the principle of the present invention, and these improvements and modifications shall fall into the protection scope of the claims of the present invention.

**Claims**

1. A block-chain based carbon trading system, comprising:

a carbon emission measurement module (10) configured to measure carbon emission of greenhouse gas produced by fossil fuel burning or gas leakage in the fields of energy, industry and/or transportation to generate carbon emission data;
a block chain carbon emission checking module (20) configured to receive the carbon emission data generated by the carbon emission measurement module (10) and add a timestamp onto the carbon emission data to generate carbon emission information;

8

a carbon emission reduction calculation module (30) configured to measure carbon emission reduction of reduced greenhouse gas in the fields of energy, industry and/or transportation after green energy and energy-saving measures are adopted to generate carbon emission reduction data; and

a block chain carbon emission reduction checking module (40) configured to receive the carbon emission reduction data generated by the carbon emission reduction calculation module (30) and add a timestamp onto the carbon emission reduction data to check the generated carbon emission reduction information.

2. The system of claim 1, wherein the carbon emission measurement module (10) comprises a carbon dioxide monitoring module and a comprehensive carbon dioxide emission monitoring and calculation module;

the carbon dioxide monitoring module is configured to monitor a carbon dioxide concentration of the flue gas, and transmit the obtained carbon dioxide concentration data to the comprehensive carbon dioxide emission monitoring and calculation module; and

the comprehensive carbon dioxide emission monitoring and calculation module is configured to calculate carbon dioxide emission data of the flue gas according to the received carbon dioxide concentration data and pre-monitored total flue gas flow data.

3. The system of claim 1, wherein the carbon emission measurement module (10) further comprises module for monitoring other greenhouse gases and a carbon dioxide conversion module;

the module for monitoring other greenhouse gases is configured to monitor a concentration of other greenhouse gases except carbon dioxide in the flue gas, and transmit the obtained concentration data of other greenhouse gases to the carbon dioxide conversion module;

the carbon dioxide conversion module is configured to obtain equivalent carbon dioxide concentration data through conversion according to the concentration data of other greenhouse gases, and transmit the converted carbon dioxide concentration data to the comprehensive carbon dioxide emission monitoring and calculation module; and

the comprehensive carbon dioxide emission monitoring and calculation module is configured to calculate carbon emission data of the flue gas according to the received carbon dioxide concentration data, pre-monitored total flue gas flow data and the converted carbon dioxide concentration data.

4. The system of claim 2, wherein the carbon dioxide monitoring module is connected to a flue gas on-line sampling device to monitor a carbon dioxide concentration of the flue gas through at least one of a non-dispersive infra-red (NDIR) analysis method, a tunable diode laser absorption spectroscopy (TDLAS) method, an infrared spectroscopy method, a gas sensitive electrode method, a gas chromatography method, a gas filter monitoring method, and a wavelength scanning-cavity ring-down spectroscopy (WS-CRDS) method.

5. The system of claim 2, wherein the module for monitoring other greenhouse gases is connected to the flue gas on-line sampling device to monitor a concentration of other greenhouse gases except carbon dioxide in the flue gas through at least one of a non-dispersive infra-red (NDIR) analysis method, a tunable diode laser absorption spectroscopy (TDLAS) method, an infrared spectroscopy method, a gas sensitive electrode method, a gas chromatography method, a gas filter monitoring method, and a wavelength scanning-cavity ring-down spectroscopy (WS-CRDS) method.

6. The system of claim 3, wherein the module for monitoring other greenhouse gases comprises a methane monitoring component, a nitrous oxide monitoring component and/or a fluoride monitoring component.

7. The system of claim 3, wherein the carbon dioxide conversion module adopts at least one of a global warming potential (GWP) conversion method, a global temperature change potential (GTP) conversion method and a comprehensive conversion method to convert the concentration of other greenhouse gases into a carbon dioxide concentration.

8. The system of claim 7, wherein the comprehensive conversion method is based on the following equation (1) for calculation:

$$GWP_{corrected} = A*GWP + B*GTP \qquad \text{Equation (1)};$$

$GWP_{corrected}$ represents a carbon dioxide concentration converted through the comprehensive conversion method; GWP represents a carbon dioxide concentration converted through the GWP conversion method; the GTP represents a carbon dioxide concentration converted through the GTP conversion method; and A and B represent predetermined correction coefficients, respectively.

9. The system of claim 3, wherein the module for monitoring other greenhouse gases comprises one or more of a methane monitoring module, a nitrous oxide monitoring module, a hydrofluorocarbon monitoring module, a perfluorocarbon monitoring module, a hexafluoroethane monitoring module and a nitrogen trifluoride monitoring module.

10. The system of claim 1, wherein the block chain carbon emission checking module (20) is further configured to add a timestamp onto carbon emission data and a carbon emission right of each greenhouse gas emission unit in the fields of energy, industry and transportation, and record the timestamp in the block chain, the carbon emission data being from the carbon emission measurement module (10). Comparing the quota or standard of carbon emission load with the actual carbon emission load, factories can purchase the carbon emission right if they produce carbon emissions beyond the threshold they are allocated and they can also sell the carbon emission right if they reduce the carbon emissions below the threshold they are allocated.

11. The system of claim 1, wherein the block chain carbon emission checking module (20) adopts a smart contract to automatically determine carbon emission right consumption arising from the carbon emission of each greenhouse gas emission unit. The block chain carbon emission checking module (20) is further configured to complete carbon emission right trading among different greenhouse gas emission units or between a greenhouse gas emission unit and a carbon emission reduction unit, trading information generated during each carbon emission right trading being recorded in the block chain and being non-falsifiable.

12. The system of claim 16, wherein the carbon emission reduction units comprise related units capable of reducing carbon emissions and summarized based on a CDM methodology of the UNFCCC.

13. The system of claim 1, wherein the block chain carbon emission checking module (20) is distributed in each node of a block chain, and is capable of automatically levy a fine on a greenhouse gas emission unit whose carbon emission exceeds a preset carbon emission.

14. The system of claim 2, wherein the flue gas comes from a fossil fuel boiler in at least one of a large-scale thermal power plant, a large-scale steel-making plant, a large-scale coking plant, a large-scale cement plant and a large-scale chemical plant.

15. The system of claim 1, wherein the block chain carbon emission checking module (20) is provided with a data layer for storing the following data:

carbon emission data measured by the carbon emission measurement module (10);
carbon emission right trading data which is all peer-to-peer carbon emission right trading data in the block chain;
a carbon purchasing and selling identifier which is a binary digit character string for distinguishing a carbon emission right purchaser who produce carbon emissions beyond the allocated threshold from a carbon emission right seller who reduce carbon emissions below the allocated threshold, the first binary digit character string in the carbon purchasing and selling identifier representing that a current block chain node is the carbon emission right purchaser, and the second binary digit character string in the carbon purchasing and selling identifier representing that a current block chain node is the carbon emission right seller; and
a timestamp for recording the peer-to-peer carbon emission right trading time in a block chain network; and
the data layer is further configured to add a timestamp onto carbon emission right trading data of each node to form a data block which is recorded in the block chain and is non-falsifiable.

10       20       40       30

| Carbon Emission Measurement Module | Block Chain Carbon Emission Checking Module | Carbon Emission Reduction Calculation Module | Block Chain Carbon Emission Reduction Checking Module |

FIG. 1

10       20       40       30

| The Field of Transportation | Road; Vehicle Emissions | Carbon Emission Measurement Module | Block Chain Carbon Emission Checking Module | | Carbon Emission Reduction Calculation Module | Block Chain Carbon Emission Reduction Checking Module |
| | Aviation; Aircraft Emissions | Carbon Emission Measurement Module | Block Chain Carbon Emission Checking Module | | Carbon Emission Reduction Calculation Module | Block Chain Carbon Emission Reduction Checking Module |
| | Emissions of Railway Locomotives, Ships, and Other Vehicles | Carbon Emission Measurement Module | Block Chain Carbon Emission Checking Module | | Carbon Emission Reduction Calculation Module | Block Chain Carbon Emission Reduction Checking Module |

FIG. 2

10       20       40       30

| The Field of Energy | Electrical Power | Carbon Emission Measurement Module | Block Chain Carbon Emission Checking Module | | Carbon Emission Reduction Calculation Module | Block Chain Carbon Emission Reduction Checking Module |
| | Thermal Power; Heating Boiler | Carbon Emission Measurement Module | Block Chain Carbon Emission Checking Module | | Carbon Emission Reduction Calculation Module | Block Chain Carbon Emission Reduction Checking Module |

FIG. 3

| | 10 | 20 | | 40 | 30 |
|---|---|---|---|---|---|

| The Field of Industry | Iron and Steel Industry; Furnace Boiler | Carbon Emission Measurement Module | Block Chain Carbon Emission Checking Module | | Carbon Emission Reduction Calculation Module | Block Chain Carbon Emission Reduction Checking Module |
| | Cement Industry; Boiler | Carbon Emission Measurement Module | Block Chain Carbon Emission Checking Module | | Carbon Emission Reduction Calculation Module | Block Chain Carbon Emission Reduction Checking Module |
| | Gas Leaked in Petroleum Refining | Carbon Emission Measurement Module | Block Chain Carbon Emission Checking Module | | Carbon Emission Reduction Calculation Module | Block Chain Carbon Emission Reduction Checking Module |
| | Other Industry Boilers | Carbon Emission Measurement Module | Block Chain Carbon Emission Checking Module | | Carbon Emission Reduction Calculation Module | Block Chain Carbon Emission Reduction Checking Module |

FIG. 4

10

**Carbon Emission Measurement Module**

101

**Carbon Dioxide Monitoring Module**

103

**Module for Monitoring Other Greenhouse Gases**

104

**Carbon Dioxide Conversion Module**

Sampled Gas

102

**Comprehensive Carbon Dioxide Emission Monitoring and Calculation Module**

FIG. 5

**Greenhouse Gas Concentration Detection Methods**

Sampled Gas

**Non-dispersive Infra-red (NDIR) Analysis Method**

**Tunable Diode Laser Absorption Spectroscopy (TDLAS) Method**

**Infrared Spectroscopy Method**

**Gas Sensitive Electrode Method**

**Gas Chromatography Method**

**Gas Filter Monitoring Method**

**Wavelength Scanning-Cavity Ring-down Spectroscopy (WS-CRDS) Method**

**Comprehensive Carbon Dioxide Emission Monitoring and Calculation Module**

FIG. 6

104

Carbon Dioxide Conversion Module

Global Warming Potential (GWP) Conversion Method

Global Temperature Change Potential (GTP) Conversion Method

Comprehensive Conversion Method

FIG. 7

Europäisches
Patentamt

European
Patent Office

Office européen
des brevets

# EUROPEAN SEARCH REPORT

Application Number

EP 18 18 3285

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/088657 A2 (PLANETARY EMISSIONS MAN [US]; MARINO BRUNO D V [US]) 5 August 2010 (2010-08-05) * page 15, paragraph 55 - page 16, paragraph 65 * * page 24, paragraph 102 - page 105, paragraph 309 * | 1-15 | INV. G06Q40/04 G06Q10/06 |
| A | US 2017/083907 A1 (MCDONOUGH JOHN C [US] ET AL) 23 March 2017 (2017-03-23) * page 4, paragraph 88 * | 1-15 | |
| A | US 2017/103468 A1 (ORSINI LAWRENCE [US] ET AL) 13 April 2017 (2017-04-13) * page 4, paragraph 37 * * page 5, paragraph 63 - page 5, paragraph 73 * | 1-15 | |

TECHNICAL FIELDS
SEARCHED (IPC)

G06Q

The present search report has been drawn up for all claims

| Place of search | Date of completion of the search | Examiner |
|---|---|---|
| The Hague | 11 September 2018 | Rachkov, Vassil |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ..............................................................................
& : member of the same patent family, corresponding
    document

EPO FORM 1503 03.82 (P04C01)

EP 3 444 771 A1

## ANNEX TO THE EUROPEAN SEARCH REPORT
## ON EUROPEAN PATENT APPLICATION NO.

EP 18 18 3285

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2018

| Patent document cited in search report | | Publication date | Patent family member(s) | | Publication date |
|---|---|---|---|---|---|
| WO 2010088657 | A2 | 05-08-2010 | AU | 2010207964 A1 | 18-08-2011 |
| | | | CA | 2751209 A1 | 05-08-2010 |
| | | | CN | 102405404 A | 04-04-2012 |
| | | | CN | 107016445 A | 04-08-2017 |
| | | | EP | 2391881 A2 | 07-12-2011 |
| | | | JP | 5587344 B2 | 10-09-2014 |
| | | | JP | 5908541 B2 | 26-04-2016 |
| | | | JP | 2012516999 A | 26-07-2012 |
| | | | JP | 2014211451 A | 13-11-2014 |
| | | | KR | 20120058444 A | 07-06-2012 |
| | | | KR | 20160056949 A | 20-05-2016 |
| | | | US | 2010198736 A1 | 05-08-2010 |
| | | | US | 2014172323 A1 | 19-06-2014 |
| | | | WO | 2010088657 A2 | 05-08-2010 |
| US 2017083907 | A1 | 23-03-2017 | NONE | | |
| US 2017103468 | A1 | 13-04-2017 | CN | 108431845 A | 21-08-2018 |
| | | | EP | 3362965 A1 | 22-08-2018 |
| | | | US | 2017103468 A1 | 13-04-2017 |
| | | | WO | 2017066431 A1 | 20-04-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 20162020

**Non-patent literature cited in the description**

- **SATOSHI NAKAMOTO.** *Bitcoin: A Peer-to-Peer Electronic Cash System,* November 2008